# EUROPEAN PATENT APPLICATION

(11) **EP 2 489 369 A1**
(43) Date of publication of application: **22.08.2012**
(21) Application number: 11176964.2
(22) Date of filing: 11.09.2008
(51) Int. Cl.: A61K 45/06, A61P 31/20

(54) **A method of treating Hepatitis C patients**

(30) Priority: 14.09.2007 US 993753 P; 12.10.2007 US 998762 P; 30.10.2007 US 1494 P
(62) Divisional of application: 08831728.4
(71) Applicant: Merck Sharp & Dohme Corp., Rahway NJ 07065-0907 (US)
(72) Inventor: Albrecht, Janice K., Winter Park, FL Florida 32789 (US); Brass, Clifford A., New York, NY New York 10025 (US); Ralston, Robert Orville, II, Union, NJ New Jersey 07083 (US)
(74) Representative: Jaap, David Robert

(57) **Abstract**

The application discloses a combination for use in a method of treating patients suffering from hepatitis C infection who are treatment naive or who have relapsed from having a negative HCV status.

## Description

### Field of the Invention

This application discloses a novel method for treating patients suffering from Hepatitis C infection and a novel method for identifying patients suffering from Hepatitis C infection that will be responsive to said treatment method.

### Background of the Invention

Identification of any publication in this section or any section of this application is not an admission that such publication is prior art to the present invention.

As described in Published international application WO 2007/092616 A2, filed on February 9, 2007 (the '616 publication), which is incorporated herein in its entirety, the standard treatments of hepatitis C viral infection include treatment with interferon alpha (also referred to herein as interferon alfa) and treatment using combination therapy with ribavirin, which is an antiviral compound, and interferon alpha, for example, Peglntron® peginterferon alfa-2b, Powder for injection (from Schering Corporation, Kenilworth, NJ). As further described in the '616 publication, the addition of a HCV protease inhibitor to standard therapy can improve results, for example the addition of the compounds of Formulae Ia (and its isomers), IIa (and its isomers) and III (and its isomers).

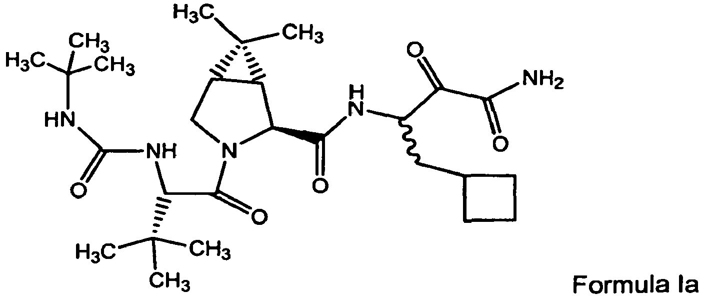

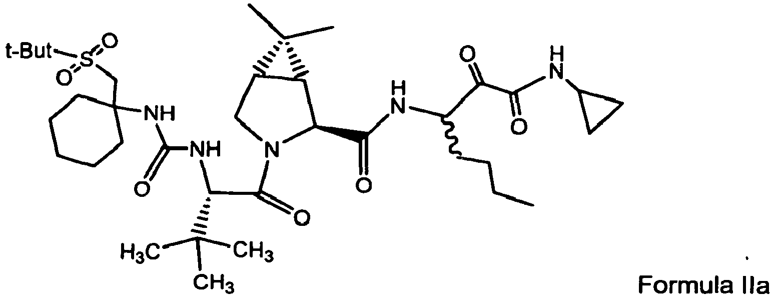

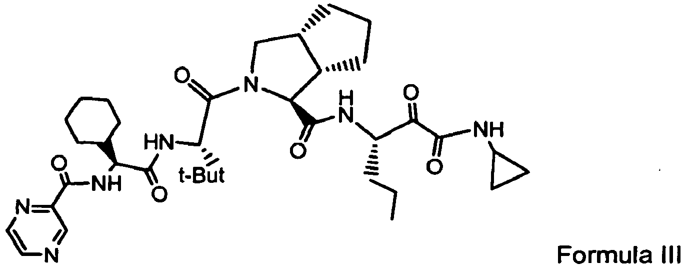

Methods of preparing the compound of Formula Ia and its related isomers are described in, for example, U.S. patent no. 7,012,066, which issued March 14, 2006 to Saskena et al., and which is incorporated herein by reference in its entirety. The '066 patent describes the preparation of the compounds of Formulae Ia, Ib, and Ic (described herein), at col. 113, Example XXIV (cols. 448 to 451) and col. 1259), incorporated herein by reference. Methods of preparing the compound of Formula IIa and its isomers are described in, for example, published U.S. patent application no. 2007/0042968, filed February 24, 2005, see claim 46 and Example 696 which illustrates the synthesis of analogous compounds, and which is incorporated herein by reference in its entirety. Formulations incorporating the compounds of Formulae Ia and IIa (and related compounds and isomers), and methods of treating HCV infections using those formulations are described in, for example, published international application nos. WO 2007/092616, applicant Schering Corporation (published August 16, 2007), which is incorporated herein by reference in its entirety. Formulations incorporating the compound of Formula III, and related compounds and isomers thereof are described in published international application no. WO 2002/18369 (the '369 publication), applicant Eli Lilly Company (published March 7, 2002), for example, compound BW on page 52 thereof, and compound CU on page 3316 thereof. The '369 publication is incorporated herein by reference in its entirety.

In spite of encouraging response in some patients when subjected to these various treatments, the response among patients infected with Hepatitis C virus is approximately 50%, particularly amongst the patients infected with genotype I HCV. Moreover, although therapy which includes combination with an HCV protease inhibitor shows improved response, there is still a low incidence of patients exhibiting a sustained virologic response (SVR), which is defined as a patient completing at least 24 weeks post treatment in a state free of viral RNA as measured in accordance with the assay methodology described in the Examples section herein. SVR is also described in detail by Dr. Steven L. Flamm in the Journal of the American Medical Association, Vol. 289, No. 18, pp. 2413 to 2417. There is also a need to provide therapy which reduces the time that patients show evidence of complete viral suppression (negative HCV status) following the initiation of treatment.

Accordingly, there remains a need for a treatment methodology which quickly brings the patients' viral load to an undetectable level, which in turn enhances in patients the achievement of a sustained viral response (SVR). There further remains a need for a treatment methodology that reduces the likelihood of developing treatment resistant strains of HCV. What is needed also is method of identifying patients having a high likelihood of successful outcome using an HCV treatment methodology that reduces the likelihood of a patient developing treatment resistant strains of HCV and increasing the likelihood of achieving an SVR.

### Objectives and Summary of the Invention

The above and other advantages are provided by the present invention which in one aspect provides a method of treating patients suffering from hepatitis C infection selected from patients that are treatment naïve and patients that have relapsed from having a negative HCV status, the method comprising: (a) administering a combination of at least one antiviral compound and an interferon for a lead-in period; (b) at the end of said lead-in period administering a combination of at least one antiviral compound, an interferon, and at least one HCV protease inhibitor compound for a second treatment period; and (c) optionally at the end of said second treatment period evaluating the patient for an SVR.

In some embodiments it is preferred to use Pegintron as the interferon throughout the treatment. In some embodiments it is preferred to use ribavarin as the antiviral compound throughout the treatment. In some embodiments it is preferred to select at least one HCV protease inhibitor from those described in issued U.S. patent no. 7012066, published U.S. patent application no. 2007/0042968, published international patent application no. WO 2007/092616, and published international patent application no. WO 2002/18369. In some embodiments it is preferred to administer, to those patients receiving at least one HCV protease inhibitor, at least one HCV protease inhibitor selected from compounds having the formula: 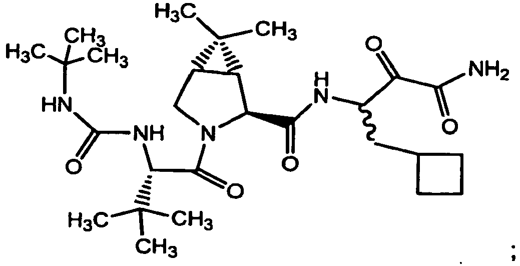 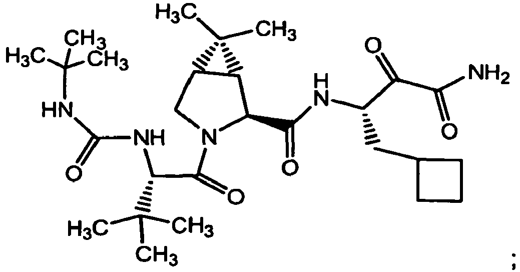 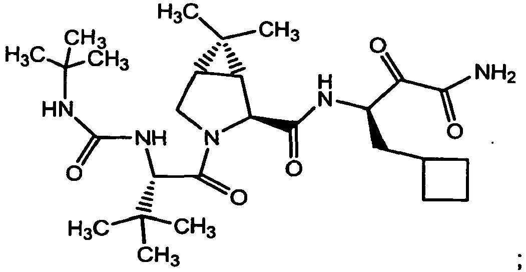 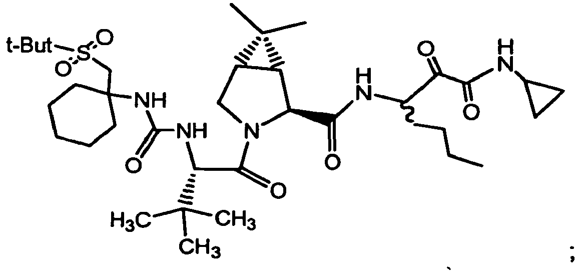 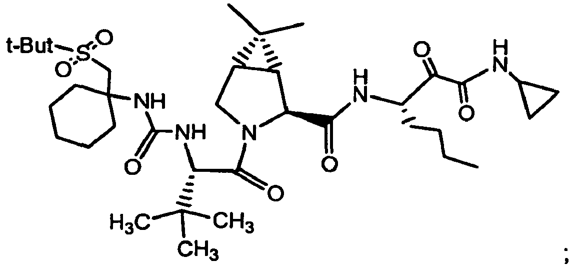 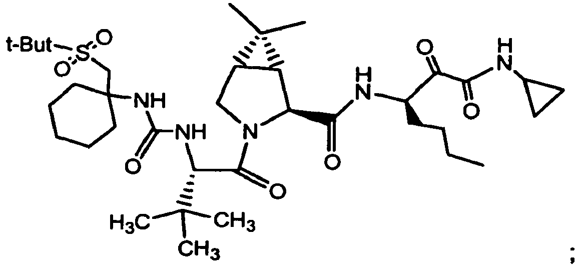 and

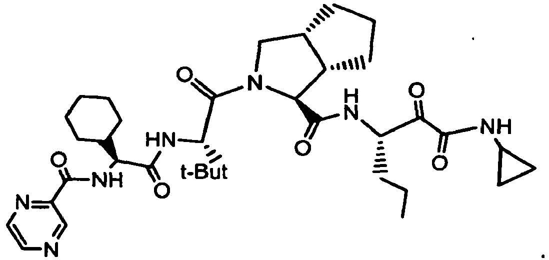

In some embodiments it is preferred for the lead-in period to have a duration of from about 2 to about 6 weeks, preferably 4 weeks. In some embodiments, it is preferred for the second treatment period to have a period of from about 24 weeks to about 48 weeks, preferably about 24 weeks. In some embodiments it is preferred for the entire treatment period, including lead-in period and second treatment period to last for about 24 weeks. In some embodiments it is preferred to carry out the second treatment period until a non-detectable level of virus is achieved and sustained in the patient. Optionally, the patient is monitored for 24 weeks following treatment to insure a non-detectable level of virus in plasma using a standard RNA detection assay, including RT-PCR based assays such as the Roche COBAS TaqMan^{®} HCV/HPS assay.

In some embodiments it is preferred to have a lead-in period lasting up to about 17 weeks, preferably from about 2 weeks to about 17 weeks, more preferably from about 8 weeks to about 17 weeks, and more preferably from about 16 to about 17 weeks.

In another aspect, the present invention provides a method of identifying among a group of treatment naive patients and relapsed patients suffering from hepatitis C infection, patients who may be successfully managed to an SVR status, the method comprising: administering a combination of at least one antiviral compound and an interferon for a lead in period; and thereafter administering a combination of at least one antiviral compound, an interferon, and at least one HCV protease inhibitor for a second treatment period if a viral load drop is observed after the lead-in period, the second period lasting until a virus-free state is achieved. In some embodiments it is preferred to select patients that have had a viral load drop of at least log 2. In some embodiments it is preferred to administer during the lead-in or screening period a combination of at least one antiviral compound and an interferon that comprises ribavirin and Pegintron.

In some embodiments it is preferred to administer during the second treatment period, in addition to at least one interferon and at least one antiviral compound, at least one HCV protease inhibitor compound. In some embodiments it is preferred to select at least one HCV protease inhibitor compound from any of the HCV protease inhibiting compounds described in U.S. Patent No. 7,012,066, published international application no. WO 2007/092616 or in published international application no. WO 2002/18369. In some embodiments it is preferred to administer to patients receiving at least one HCV protease inhibitor compound, an HCV protease inhibitor is selected from compounds having the formula: 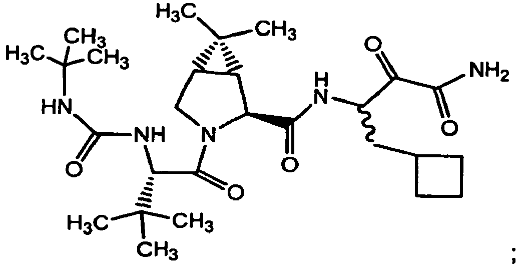 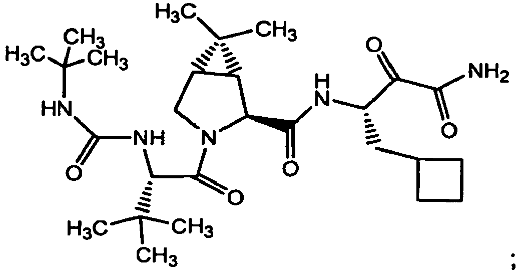 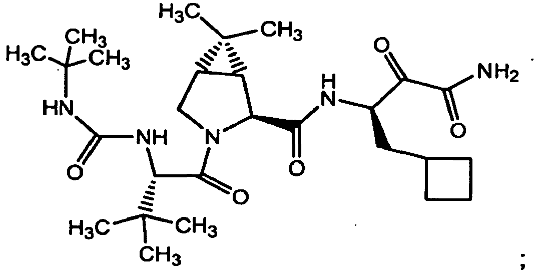 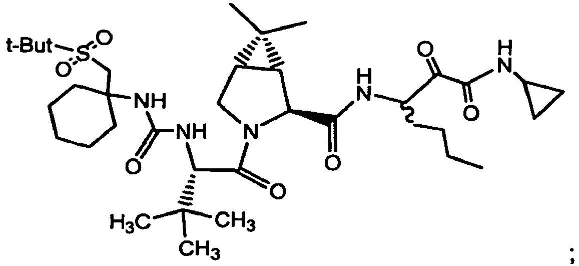 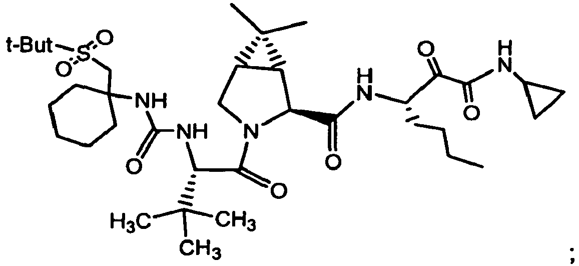 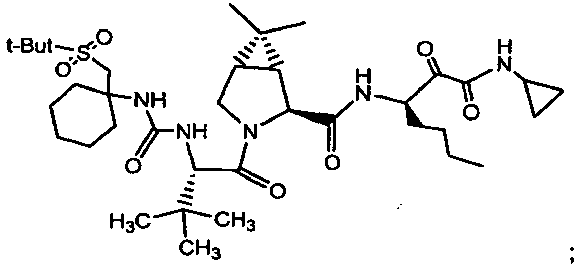 and

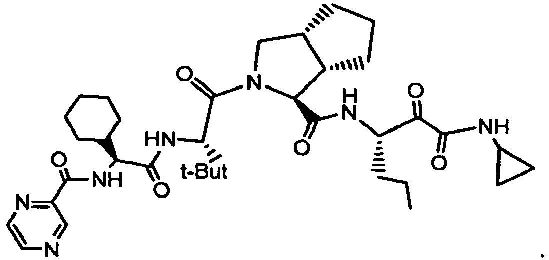

In some embodiments it is preferred to select a lead-in period for screening patients that has a duration of 4 weeks. In some embodiments it is preferred to select a second treatment period, for those patients exhibiting a suitable viral load drop during the lead-in period, that has a duration of from about 12 weeks to about 24 weeks. In some embodiments, for patients not exhibiting satisfactory viral load drop during the lead-in period, for example, a viral load drop of at least 2 log or greater, it is preferred to continue the combination treatment for 48 weeks.

In some embodiments of either the screening method or the treatment method, it is preferred to administer interferon to patients during the entire treatment cycle in an amount of from about 0.5 microgram/ Kg of patient weight to about 1.5 microgram/Kg of patient weight on a once weekly dosing schedule.

In some embodiments of either the screening method or the treatment method, it is preferred to administer at least one antiviral compound to patients during any of the time periods in which an antiviral compound is administered in an amount of from about 10 mg/Kg of patient weight to about 20 mg/Kg of patient weight with the dosage divided into 2 doses per 24 hours.

In some embodiments of the treatment method it is preferred to administer the compound of Formula Ia (boceprevir) as the HCV protease inhibitor in an amount of about 800 mg at intervals of from about 7 hours to about 9 hours during the treatment period in which it is administered.

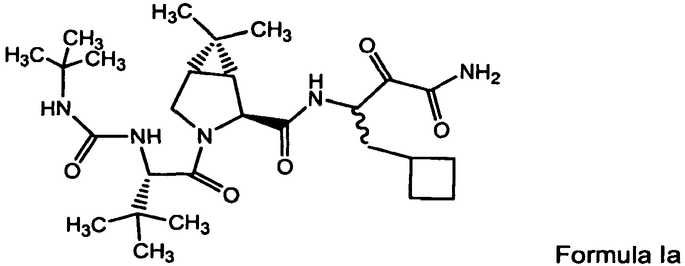

### Detailed Description of the Invention

As mentioned above, the treatment standard for HCV infection, a combination of PEG-interferon alpha conjugates and ribavirin, fails to achieve a sustained viral response (SVR) in a number of patients and thus these patients have persistent infections which lead to organ damage and failure. When treatment comprises a protease inhibitor, alone or in combination with an interferon and an antiviral compound, failure of some subjects to evidence complete response still remains an issue.

The inventors have surprisingly found that there is a correlation between the elapsed time after commencing treatment that it takes a patient to show evidence of complete viral suppression (achieve a negative HCV status) and those patients which will achieve a sustained viral response leading to a disease-free state. Accordingly, the inventors have surprisingly found that the longer it takes a patient suffering from an HCV infection, particularly HCV genotype 1 subjects, to achieve a negative HCV status after starting treatment, the less likely they are to achieve a sustained viral response (SVR). Negative HCV status is determined, for example, by having HCV-RNA levels below the limit of detection. SVR status is determined by a patient maintaining a non-detectable level of virus for 24 weeks following treatment. The inventors have surprisingly found that SVR is strongly dependent on having achieved negative HCV status at least by about week 17, preferably by about week 16, more preferably by week 12, more preferably by week 8, more preferably by week 4, and more preferably by week 2 after starting treatment.

Moreover, the inventors have surprisingly discovered that the number of patients exhibiting a sustained viral response (SVR) among patients suffering from an HCV infection is improved by adopting a treatment schedule which includes a lead-in treatment period using a combination of an interferon, for example Pegintron, and an antiviral agent, for example ribavirin, and following the lead-in treatment period, for patients which exhibit a log 2 or greater drop in viral load, a second treatment period which includes a combination of an interferon, for example Pegintron, an antiviral agent, for example, ribavirin, and an HCV protease inhibitor, for example, one or more of the compounds of Formulae Ia, Ib, Ic, IIa, IIb, IIc, III and isomers of any thereof, administered for a period sufficient to achieve an SVR.

Illustrative interferons useful in the present invention include interferon alfa-2a (Roferon®-A, from Hoffmann La-Roche, Nutley N.J.) in the form of peginterferon alfa-2a (e.g., as sold under the trade name PEGASYS®, interferon alfa-2b (INTRON^{®} A, from Schering Corporation, Kenilworth, NJ) in the form of peginterferon alfa-2b (sold under the trade name Peglntron®) and consensus interferon as defined by determination of a consensus sequence of naturally occurring interferon alphas (Infergen®, originally developed by Amgen, Thousand Oaks, Calif.). Other interferons useful in the present invention include fusions between interferon alfa and a non-interferon protein, such as Albuferon®, a fusion between human serum albumin (HSA) and interferon alfa from Human Genome Sciences, Rockville, MD. For the present invention it is preferred to use PEG-interferon alpha conjugates. PEG-interferon alpha conjugates are interferon alpha molecules covalently attached to a PEG molecule. It is preferred for Pegintron to be used as the interferon administered during the treatment method of the invention. When Pegintron is selected, it is preferred to administer it to patients on a once per week injection schedule in an amount of from about 0.5 micrograms/Kg of patient body weight to about 1.5 micrograms/Kg of patient body weight, preferably, Pegintron is injected once per week in an amount of about 1.5 micrograms/Kg of patient body weight.

For the present invention, it is preferred to use ribavirin (for example, REBETOL® from Schering Corporation) as the antiviral agent administered in the treatment, although it will be appreciated that other antiviral agents may be used. When ribavirin is used it is preferred to administer from about 10 mg/Kg of patient body weight to about 20 mg/Kg of patient body weight, preferably about 16 mg/Kg of patient body weight. In some embodiments it is preferred to divide the daily dosage into two portions administered about 12 hours apart.

For the present invention, the HCV protease inhibitor compounds may be selected from any of those described in published U.S. patent application no. 2007/0042968, filed February 24, 2005, for example, the compounds of Formulae IIa, IIb, and IIc.

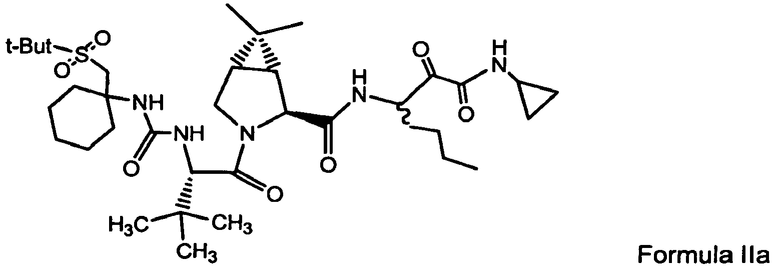

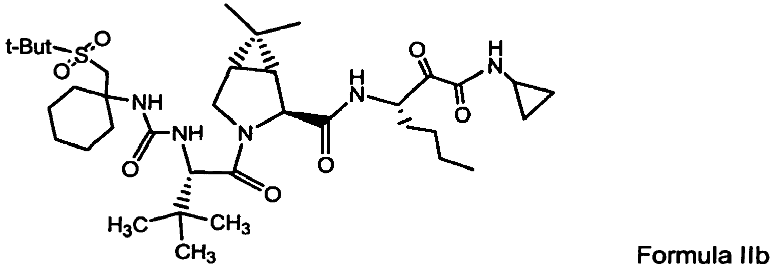

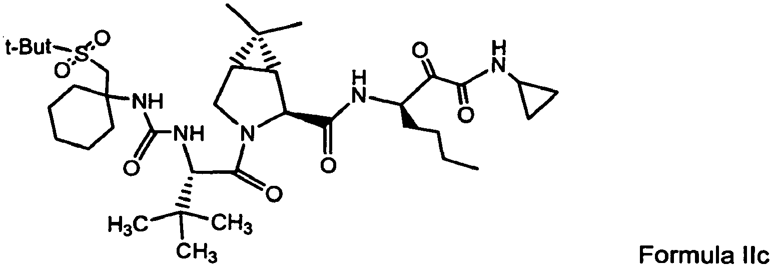

Additionally, for the present invention HCV protease inhibitor compounds may be selected from any of those described in U.S. patent no. 7,012,066, issued March 14, 2006 to Saskena et al., for example, the compounds of Formulae Ia, Ib, and Ic.

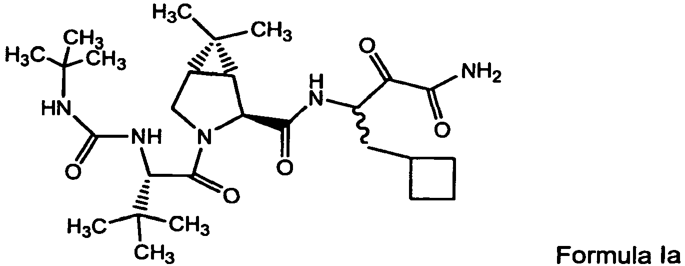

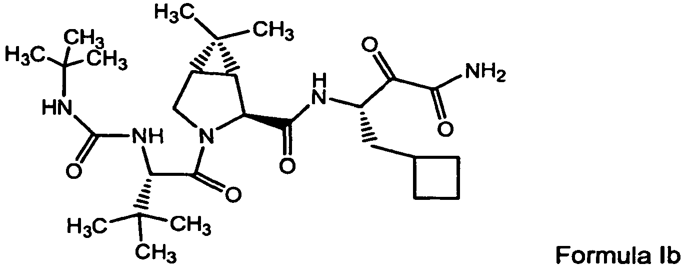

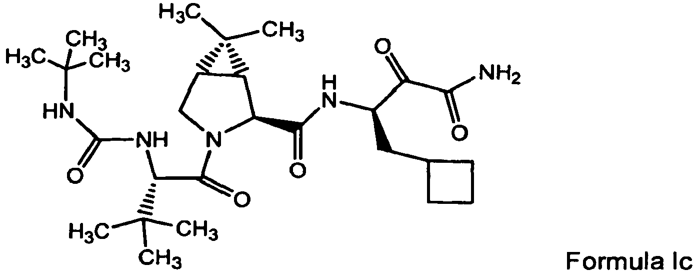

Additionally, for the present invention HCV protease inhibitor compounds may be selected from any of those described in published international application no. WO 2002/18369, applicant Eli Lilly Company, published March 7, 2002, for example, the compound of Formula III.

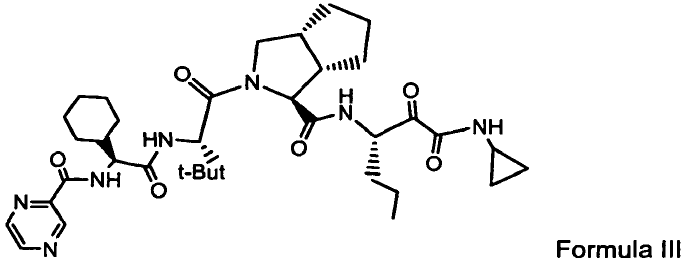

Preferably, for the present invention, the HCV protease inhibitor compound is selected from one or more compounds of Formulae Ia, Ib, Ic, IIa, IIb, IIc, and III and isomers thereof, more preferably the HCV protease inhibitor is selected from compounds of the Formulae Ia, IIb, and III. In some embodiments it is preferred to use the compound of Formula Ia as the HCV protease inhibitor compound. Although in most instances a single HCV protease inhibitor compound will be employed, in some instances multiple HCV protease inhibitor compounds may given in combination.

In some embodiments of the present method of treatment employing a compound of the Formulae la, Ib, or Ic, it is preferred to administer to a patient an amount of the HCV protease inhibitor which is greater than about 300 mg/day, more preferably greater than about 600 mg/day, more preferably greater than about 1200 mg/day, and more preferably at least about 2400 mg/day. In some embodiments it is preferred to administer the HCV protease inhibitor orally in three equal dosage amounts spaced from about 7 hours to about 9 hours between doses.

In some embodiments of the present method of treatment employing a compound of the Formulae IIa, IIb, or IIc, it is preferred to administer to a patient an amount of the HCV protease inhibitor which is from about 200 mg to about 2400 mg daily. In some embodiments it is preferred to administer three doses in 24 hours of a compound of the Formulae IIa, IIb, or IIc in an amount of from about 200 mg to about 800 mg. In some embodiments it is preferred to coadminister a once daily dose of a CYP-3 inhibitor. In some embodiments it is preferred to administer two doses in 24 hours of a compound of the Formulae IIa, IIb, or IIc in an amount of from about 100 mg to about 400 mg and to coadminister twice daily a CYP-3 inhibitor, particularly one or more compounds that inhibit cytochrome oxidase P450 3A4. An example of a suitable CYP-3 inhibitor for coadministration with a compound of Formulae IIa, IIb, or IIc is ritonavir (ABT-538) available under the NORVIR® trade name from Abbott Laboratories, Abbott Park, **IL** 60064.

In some embodiments of the present invention, it is preferred for the lead-in period prior to the beginning of treatment with a protease inhibitor compound to be from about two weeks to about 6 weeks, more preferably 4 weeks of treatment with an interferon and an anti-viral compound, for example, Pegintron and ribavirin. Longer or shorter lead-in periods can be used, for example, a lead-in period of up to 12 weeks, a lead-in period of up to 16 weeks, a lead-in period of 17 weeks duration. Alternatively, a lead-in period of from about 12 weeks to about 17 weeks duration can be used according to the present invention.

In some embodiments of the present invention, it is preferred to provide a second treatment period in which the patient is receiving an interferon, an antiviral compound, and an HCV protease inhibitor for a period of from about 12 weeks to about 25 weeks, for example, a period of 24 weeks, although longer periods, for example 48 weeks, and shorter periods can be employed.

In some embodiments, following the lead-in period, patients are evaluated to determine if they are eligible for receiving benefit from a second treatment period. In general, those patients having a drop in viral load or a negative HCV status at the end of the lead-in period will be eligible to receive benefit from a second treatment period, and accordingly treatment is initiated for a second period following the lead-in period comprising administering the combination of an interferon, an antiviral agent, and an HCV protease inhibitor for a period of time which insures an sustained viral response (SVR). In some embodiments, a patient is considered responsive, and therefore a candidate for receiving benefit from the addition of an HCV protease inhibitor during a second treatment period, if the patient demonstrates a drop in viral load of about log 2 by week 17 of a lead-in period, more preferably by week 12 lead-in period. In some embodiments, a protease inhibitor is added to the therapy, in combination with an interferon and an antiviral compound, after a lead-in period of 12 to 17 weeks.

The inventors have surprisingly found that patients exhibiting a negative HCV status within 8 to 12 weeks of the start of treatment have a much higher success rate of achieving an SVR than those patients which do not attain a negative HCV status during the lead-in treatment period. Moreover, the inventors have surprisingly found that even among a pedigreed group of interferon and ribavirin treatment experienced non-responders, a lead-in period of treatment with interferon and an antiviral agent followed by continued administration of the interferon and antiviral agent with coadministration of an HCV protease inhibitor yields viral-free responses in about 1/3 of the group with a sustained viral response rate exceeding three times that seen with interferon and antiviral combination therapy alone continued over the same treatment period. The inventors have surprisingly found also that a lead-in period of treatment with an interferon and an antiviral provides a greater number of patients achieving a negative HCV status than when a therapy comprising coadministering an interferon/an antiviral/ and a protease inhibitor is effected initially.

In some embodiments, after the lead-in period administering an interferon and antiviral therapy in combination, the second treatment period in which an HCV protease inhibitor and optionally a CYP-3A4 inhibitor is administered in combination with the interferon and antiviral therapy, is continued for an interval such that the total treatment time, including the lead-in period, is from about 12 weeks to about 28 weeks. In some embodiments it is preferred to have a lead-in treatment period with an interferon and an antiviral of from about 4 weeks to about 8 weeks. In some embodiments it is preferred to have a lead-in period lasting up to about 17 weeks, more preferably from about 12 weeks to about 17 weeks, and more preferably from about 16 weeks to about 17 weeks before adding an HCV protease inhibitor in combination with an interferon and an antiviral, although longer or shorter lead-in time periods can be employed. In some embodiments the lead-in period is followed by a second treatment period in which an HCV protease inhibitor is added to the interferon/antiviral combination therapy wherein the second treatment period has a duration of at least about 25 weeks.

Accordingly, and without wanting to be bound by theory, the inventors believe that the lead-in treatment period with an interferon and an antiviral compound significantly weakens the virus and suppresses viral mutation, and the addition of a protease inhibitor at the end of the lead-in period leads to more effective eradication of the virus, providing a greater sustained viral response in patients receiving therapy in accordance with the method of treatment of the present invention. Supporting this assertion, in a study of various combination therapies, a control group comprising 48 patients received 48 weeks of therapy comprising Pegintron (1.5 micro-grams/Kg weekly) and REBETOL® (ribavarin available from Schering-Plough Corporation) (800 mg to 1200 mg daily). Among these patients, at the end of therapy, only 8% were virus free, and at the end of 24 weeks following therapy, only 2% (1 patient) showed a sustained viral response. The group of patients who were non-responders from this control group (44 patients) were then administered a therapy comprising a lead-in period of 17 weeks of 1.5 micrograms/Kg of Pegintron weekly and from about 800 to about 1200 mg/day of REBETOL (from about 10 mg/Kg to about 20 mg/Kg in two equal doses every 24 hours), followed by 25 additional weeks of the same levels of Pegintron and REBETOL therapy with the addition of 800 mg of the compound of Formula Ia, administered three times a day throughout the 25 weeks. The results of this study are presented in Table I below.

**TABLE I**

| Treatment | Duration of Treatment (weeks) | HCV neg (at end of treatment) | SVR (24 weeks post-treatment) | Relapse Rate |
|---|---|---|---|---|
| Interferon/Antiviral | 48 | 8% (4/49) | 2% (1/49) | 67% (2/3) |
| **17 weeks** interferon/antiviral | 42 weeks total | 32% (14/44) | 7% (3/44) | 79% (11/14) |
| **25 weeks** interferon/antiviral/ HCV protease inhibitor | | | | |

Noteably, of the 44 non-responding patients treated with HCV protease inhibitor therapy following a lead-in period, at the end of treatment 32% (14 patients) were HCV negative. Twenty-four weeks after the end of therapy, 7% (3 patients) exhibited a sustained viral response. This is remarkable in view of the fact that none of these patients exhibited a sustained viral response after the previous 48 weeks of interferon/antiviral treatment alone. Moreover, a higher percentage of this group of non-responders attained HCV negative status after the treatment regime which included coadministration of an HCV protease inhibitor, 32% compared to about 6% after 48 weeks of interferon/antiviral treatment alone. Additionally, when a group of patients (226) receiving a therapy comprising an interferon, an antiviral, and an HCV protease inhibitor without a lead-in period were compared with a group patients (206) receiving an interferon and an antiviral for a 12 week lead-in period before receiving therapy comprising administration of an interferon, an antiviral, and an HCV protease inhibitor, it was found that only 2.9% of the group subjected to a lead-in period exhibited viral variants (as determined by HCV-RNA sequencing sensitive to a 10-20% viral population) compared to 6.6% of the group which had received no lead-in therapy.

These data support the inventors' discovery that a lead-in period with interferon and an antiviral followed by the addition of an HCV protease inhibitor yields improved results over the standard of care which is the administration of a combination therapy consisting of an interferon and an antiviral agent.

There follows examples which illustrate the treatment method of the invention.

### Example 1: Screening and Treatment Method for Hepatitis C Infected Relapsed or Treatment Naïve Patients

In a study of patients infected with HCV, a group of patients consisting of treatment naive and relapsed patients were divided into three arms, 104 patients were treated by administering a combination of Pegintron and ribavirin, 226 patients were treated by administering a combination of Pegintron, ribavirin, and boceprevir (the compound of Formula Ia) and 206 patients were treated by administering a combination of Pegintron and ribavirin for a 2 week lead-in period, a combination of Pegintron, ribavirin, and boceprevir for a first treatment period of 8 weeks, and qualified participants in the third arm will continue to receive a combination of Pegintron, ribavirin, and boceprevir for an additional 14 weeks.

During this study, all patients treated with Pegintron were treated at a dosage level of 1.5 micrograms/Kg of body weight by injection once per week throughout the treatment period. All patients receiving ribavirin were administered ribavirin at a dosage level of 16 mg/Kg of body weight (800 mg to1400 mg) per day divided into two oral each day throughout the treatment period. All patients receiving broceprevir were given 800 mg every 7 to 9 hours throughout the treatment period.

The results of those patients receiving treatment are reported in Table II, below. Table II indicates that a greater number of patients treated achieve negative HCV status in a shorter period of treatment than for the other treatment methods. It will be found that a significantly greater number of treatment naïve and relapsed patients suffering from HCV infection in the lead-in treatment group which show earlier evidence of a negative HCV status, in this study, by having HCV-RNA levels below the limit of detection, will experience a sustained viral response than will be observed in the other two treatment groups among persons showing evidence of a negative HCV status.

**Table II**

| Treatment arm | % of patients presenting negative HCV status / cumulative total | | | | | | |
|---|---|---|---|---|---|---|---|
| Weeks of Treatment | Lead-In (LI) = 4 weeks* | 2+LI | 4+LI | 6+LI | 8+LI | 10+LI | 12+LI |
| Lead In followed by interferon/antiviral/ boceprevir | 6% | 35% / 41% | 23% 64% | 10% / 74% | 5% / 79% | | |
| Interferon/antiviral | **No lead-in** | 4% | 4% / 8% | 9% / 17% | 9% / 26% | 8% / 34% | 7% / 41% |
| interferon/antiviral/ boceprevir | **No lead-in** | 11% | 27%/ 38% | 19% / 57% | 10% / 67% | 8% / 75% | 2% / 77% |
| | | | | | | | |

| | | | | | | | |
|---|---|---|---|---|---|---|---|
| *The other treatment arms use no lead-in period, thus treatment commences two weeks later than in the first treatment arm. | | | | | | | |

The screening procedure used to determine a negative HCV status follows.

### Standard Operating Procedure for Quantitative RT-PCR

### A Principle

The HCV-RNA copies are determined by extracting total RNA from sample and performing the reverse transcription-polymerase chain reaction (RT-PCR). The RT-PCR used is an automated method that allows for real-time quantitation of target nucleic acid molecules. This method utilizes the reverse transcriptase, 5'-exonuclease and DNA polymerase activities of the rTth DNA polymerase. The rTth DNA polymerase first makes DNA copies of the viral RNA (reverse transcriptase activity) and then proceeds to make copies of the DNA (polymerase activity). As the amplification proceeds the 5'-exonuclease activity of rTth DNA polymerase digests a sequence-specific probe. This action releases a fluorescent signal allowing quantitation of the input RNA copies.

HCV genotype is determined at SPRI by sequencing the PCR amplified DNA fragment of the 5'-untranslated region of the HCV genome. The sequence is then aligned with the published sequences of the HCV genotypes to arrive at a determination.

### B Extraction of RNA from Sample

Total RNA is extracted in an automated high throughput liquid handler and QIAamp 96 Viral RNA extraction kit from QIAGEN. This method gives high quality RNA suitable for RT-PCR.

### Quantitative RT-PCR for HCV

One-step RT-PCR is performed using rTth DNA polymerase. Direct detection of the RT-PCR product is accomplished by monitoring the increase in fluorescence of the dye-labeled probe. During PCR, if the target of interest is present, the probe specifically anneals to the target. The 5'-exonuclease activity of the rTth DNA polymerase digests the probe releasing fluorescence. This process occurs in every cycle during PCR and does not interfere with the exponential accumulation of product. The increase in fluorescence (proportional to the amount of PCR product accumulated) is detected only if the target sequence is complementary to the probe and is amplified during PCR. Because of these requirements, nonspecific amplification is not detected.

The system is able to measure PCR products after every cycle of amplification. Initial copy number of the target template is determined by analyzing the cycle-to-cycle change in fluorescence signal (.Rn) as a result of the amplification of template during PCR. The fewer cycles it takes to reach a detectable level of fluorescence (reported as Ct, the threshold cycle), the greater the initial copy number. The Sequence Detection application determines initial copy numbers of unknowns by interpolation on a standard curve generated from standards of known initial copy number.

### D Quality Control/Quality Assurance

An internal RNA control is added to each sample to check efficiency of RNA extraction and RT-PCR. Different dilutions of a precalibrated HCV control RNA is run in every assay to generate a standard curve. HCV Proficiency Panel Members are run with each assay as positive controls. Normal human sera and water are run as negative control for RNA extraction and RT-PCR.

RT-PCR HCV-RNA determinations performed by SPRI utilizing quantitative RT-PCR have been validated against WHO International Standards for hepatitis C virus RNA and the HCV Panel from Acro Metrix. The lower limit of quantitation for this assay is 29 international units/mL (IU/ mL). All HCV-RNA results will be reported in IU/mL.

### Comparative Example: Treatment of Patients Infected with Hepatitis C using VX-950

For comparison, Vertex reports that treatment with VX-950 protease inhibitor alone results in 79% of the treated patients showing evidence of negative HCV status by week four.

### Example 2: Screening and Treatment Method for Genotype 1 Hepatitis C Infected Treatment-Naïve Patients

The effectiveness of a lead-in period with interferon and ribavirin combination therapy prior to the addition of an HCV protease inhibitor was further evaluated in a study of 595 treatment-naïve patients infected with HCV genotype 1. In this study, boceprevir (compound of Formulat 1a) is being evaluated, compared to a control of Peglntron® (peginterferon alfa-2b) at 1.5 mcg/kg QW plus REBETOL® (ribavirin, USP) (800-1400 mg/day) for a treatment period of 48 weeks, in three treatment regimens:
o P/R Lead-In + B/P/R:
   ■ Pegintron (1.5 mcg/kg QW) in combination with REBETOL (800-1400 mg/day) during a first treatment period of 4 weeks followed by adding boceprevir (800 mg TID) to the combination during a second treatment period of 24 or 44 weeks; thus, the total treatment time with Pegintron and REBETOL was 28 or 48 weeks;
o B/P/R:
   ■ boceprevir (800mg TID) in combination with Pegintron (1.5 mcg/kg QW) plus REBETOL (800-1400 mg/day) for a single treatment period of 28 or 48 weeks; and
o B/P/Low-dose R:
   ■ boceprevir (800 mg TID) in combination with Pegintron (1.5 mcg/kg QW) plus low-dose REBETOL (400-1000 mg/day) for a single treatment period of 48 weeks.

The patients in this study were 77% US, 16% Black, 7% cirrhotic and 89% had high viral load (>600,000 IU/mL HCV) prior to therapy. Throughout the above described treatment periods, Pegintron was administered at a dosage level of 1.5 micrograms/kg of body weight by injection once per week, REBETOL was administered in two oral doses at a dosage level of 800-1400 mg/day or 400-1000 mg/day based on patient weight, and broceprevir was administered orally at 800 mg every 7 to 9 hours.

Plasma HCV RNA levels were measured using the Roche COBAS TaqMan^{®} HCV/HPS assay (Roche Molecular Systems, Somerville, NJ).

Results of an interim analysis for the first two treatment regimens are shown in Table III below.

**Table III: Phase II Study of Treatment-naïve Patients.**

| | Sustained Virologic Response ITT^{a} | | | | | |
|---|---|---|---|---|---|---|
| Duration of Treatment | All | RVR^{b} (Wk 4) | EVR^{c} (Wk 12) | Relapse | Viral Break-through^{d} | DC^{e} for AE |
| P/R 4 week Lead-In + B/P/R 24 wks | 56% | 82% (54/66) | 68% (58/85) | 24% | 4% | 15% |
| B/P/R 28 wks | 55% | 74% (32/43) | 69% (58/85) | 28% | 7% | 11% |
| P/R 4 week Lead-In + B/P/R 44 wks | 74% | 92% (61/66) | 89% (76/85) | 3% | 5% | 9% |
| B/P/R 48 wks | 66% | 82% (31/38) | 83% (67/81) | 7% | 11% | 19% |
| P/R Control 48 wks | 38% | 100% (8/8) | 76% (28/37) | 24% | 0% | 8% |

| | | | | | | |
|---|---|---|---|---|---|---|
| a ITT (Intention-To-Treat) analysis includes any patient who has taken at least one dose of any study drug. b Patients who achieved an SVR after achieving RVR (undetectable plasma HCV RNA) at week 4 of treatment with the combination of boceprevir, Pegintron and REBETOL or at week 4 of treatment with the Peglntron/REBETOL Control. c Patients who achieved an SVR after achieving EVR (at least a 2 log drop in plasma HCV RNA levels) at week 12 of treatment with the combination of boceprevir, Pegintron and REBETOL or at week 12 of treatment with the Peglntron/REBETOL Control. d Patients who had detectable plasma HCV RNA during treatment after achieving negative RNA status e Patients who discontinued treatment due to adverse events | | | | | | |

Addition of boceprevir to Peglntron/REBETOL combination therapy markedly increased SVR with the 28 and 48 week regimens compared to the Peglntron/REBETOL control. SVR was higher with a 4-week Peglntron/REBETOL lead-in for the 48 week regimen, while a decrease in viral breakthrough was observed with both 28 and 48-week lead-in regimens. As with the control regimen, rapid virologic response (RVR) and early virologic response (EVR) were highly predictive of response to treatment with the combination of boceprevir/Peglntron/REBETOL. The most common adverse events reported in the boceprevir regimens were fatigue, anemia, nausea and headache. Incidence of rash-related AEs was similar in boceprevir-containing regimens and the control regimen. Treatment discontinuations due to adverse events were between 9 to 19% for patients in boceprevir-containing regimens, compared to 8% in the control.

The data from this study support a therapeutic regimen for genotype 1, treatment-naïve HCV patients that includes a first treatment period of 4 weeks treatment with a two agent combination of a pegylated interferon alfa and ribavirin followed by a second treatment period of 24 to 44 weeks treatment with a three agent combination of an HCV protease inhibitor, a pegylated interferon alfa and ribavirin.

The above description of the invention is intended to be illustrative and not limiting. Various changes or modifications in the embodiments described herein may occur to those skilled in the art. These changes can be made without departing from the scope or spirit of the invention

## Claims

1. A composition comprising at least one HCV protease inhibitor compound for use in a method of treating a patient suffering from hepatitis C infection who was treatment naive or had relapsed from having a negative HCV status, and was then treated with ribavirin and an interferon for a lead-in period, wherein the method comprises the step of administering at the end of said lead-in period a combination of ribavirin, an interferon, and the HCV protease inhibitor compound for a second treatment period of sufficient duration to achieve a non-detectable viral load using HCV-RNA assay.

2. A composition comprising an interferon for use in a method of treating a patient suffering from hepatitis C infection who is treatment naive or who has relapsed from having a negative HCV status, wherein the method comprises the steps of (a) administering a combination of ribavirin and an interferon for a lead-in period; (b) at the end of said lead-in period administering a combination of ribavirin, an interferon, and at least one HCV protease inhibitor compound for a second treatment period of sufficient duration to achieve a non-detectable viral load using HCV-RNA assay.

3. The composition of any of claims 1-2, wherein the interferon administered in the lead-in period is pegylated interferon alfa.

4. The composition of any of claims 1 to 3, wherein the lead-in period has a duration of from 2 weeks to 17 weeks.

5. The composition of claim 4, wherein the lead-in period is four weeks.

6. The composition of claim 5, wherein the second treatment period has a duration of from 12 weeks to 28 weeks.

7. The composition of any of claim 1 to 6, wherein the amount of interferon administered in the lead-in period and/or the second treatment period is from 0.5 microgram/Kg of patient weight to 1.5 microgram/Kg of patient weight.

8. The composition of claim 7, wherein the amount of ribavirin administered is from 10 mg/Kg of patient weight to 20 mg/Kg of patient weight per day.

9. The composition of any one of the preceding claims, wherein the HCV protease inhibitor compound is the compound of Formula la (boceprevir)

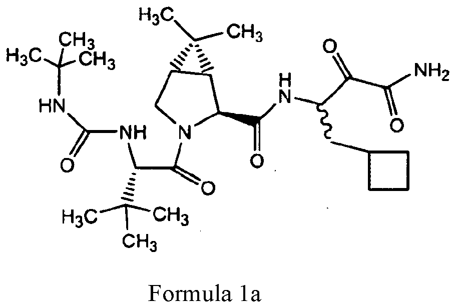

10. The composition of claim 9, wherein the amount of boceprevir administered is 800 mg at intervals of from 7 hours to 9 hours.

11. The composition of any one of the preceding claims, wherein the HCV protease inhibitor compound is the compound of Formula III.

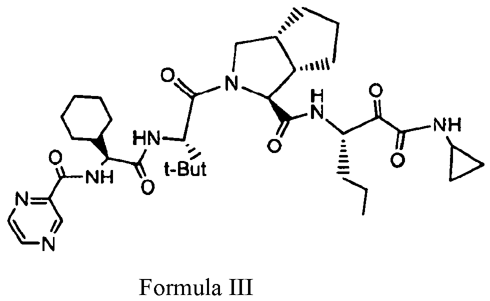

12. The composition of claim 1 or claim 2, wherein the HCV protease inhibitor is co-administered with a CYP-3A4 inhibitor.

13. The composition of claim 12, wherein the CYP-3A4 inhibitor is ritonavir.
